Europäisches Patentamt

**European Patent Office**

**Office européen des brevets**

(11) Veröffentlichungsnummer: **0 060 480**
A1

(12) **EUROPÄISCHE PATENTANMELDUNG**

(21) Anmeldenummer: **82101819.9**

(22) Anmeldetag: **08.03.82**

(51) Int. Cl.³: **A 61 B 17/36, B 01 L 3/02**

(30) Priorität: **18.03.81 DE 3110401**

(43) Veröffentlichungstag der Anmeldung: **22.09.82**
**Patentblatt 82/38**

(84) Benannte Vertragsstaaten: **AT BE FR GB IT NL**

(71) Anmelder: **MESSER GRIESHEIM GMBH, Patentabteilung Hanauer Landstrasse 330, D-6000 Frankfurt/Main 1 (DE)**

(72) Erfinder: **Klipping, Gustav, Prof. Dr., Limastrasse 38, D-1000 Berlin 37 (DE)**

(54) **Vorrichtung zur Handhabung und Dosierung kleiner Mengen tiefkalten flüssigen Mediums.**

(57) Zur Handhabung und Dosierung kleiner Mengen tiefkalten flüssigen Mediums, insbesondere Stickstoffs, beispielsweise zur Kältebehandlung von Haut- und Gewebeteilen oder in der Fertigungstechnik zum Schrumpfen, wird eine Pipette (1) mit einem Vakuumisoliermantel (14) umgeben, der ganz oder teilweise verspiegelt sein kann.

EP 0 060 480 A1

- 1 -

MESSER GRIESHEIM GMBH                    MG 1262

Kennwort: Cryo-Pipette                   EM -
Erfinder: Prof. Klipping                 Ordner: A

## Vorrichtung zur Handhabung und Dosierung kleiner Mengen tiefkalten flüssigen Mediums

Die Erfindung betrifft eine Vorrichtung zur Handhabung und Dosierung kleiner Mengen tiefkalten flüssigen Mediums, vorzugsweise flüssigen Sickstoffs, beispielsweise in der Medizin zur Kältebehandlung von Haut bzw. Gewebeteilen, in der Fertigungstechnik zum Schrumpfen kleiner Teile, zum Schnellgefrieren kleiner Objekte oder zum Einblasen der tiefkalten Flüssigkeit in Hohlkörper.

Es ist allgemein bekannt, zur Hautbehandlung, vor allem zur Entfernung von Warzen, mit flüssigem Stickstoff getränkte Wattebäusche zu verwenden. Durch das Tiefgefrieren werden gute Ergebnisse bei der Abtötung der erkrankten Gewebeteile erzielt, jedoch hat die Anwendung der stickstoffgetränkten Wattebäusche den Nachteil, daß eine genaue Abgrenzung der behandelten Hautstelle nicht möglich ist, so daß auch gesunde Gewebeteile in Mitleidenschaft gezogen werden. Außerdem ist die Tiefenwirkung zu gering und nicht kontrollierbar, und der Stickstoffverbrauch ist sehr hoch.

Bessere Ergebnisse werden in der Dermatologie mit in flüssigem Stickstoff tiefgekühlten Metallstäben erzielt, die bis zu einem Durchmesser von etwa 10 mm gut zu handhaben sind. Sie haben jedoch den Nachteil, daß größere Haut/Gewebeflächen, bspw. Warzenbeete oder flächige Tumore, nicht - wie an sich wünschenswert - in einem einzigen Kühlvorgang behandelt werden können. Darüber hinaus ist die Behandlung von Tumoren, bei der eine erhebliche Tiefenwirkung erforderlich ist, mit derartigen Metallstäben auch nicht möglich. Der Verbrauch ist naturgemäß auch relativ hoch.

Schließlich ist es auch bekannt, zur Behandlung von Hauterkrankungem mit einem Sprühgerät Kohlensäureschnee aufzusprühen. Auch hierbei ist jedoch die zu geringe Tiefenwirkung und die Gefährdung benachbarter gesunder Haut-Gewebepartien nachteilig.

In der Fertigungstechnik ist es zur Herstellung von Schrumpfverbindungen üblich, das einzusetzende Teil durch Eintauchen in flüssigen Stickstoff abzukühlen, während das aufnehmende Teil erwärmt wird. Das Eintauchen von auf Raumtemperatur befindlichen Objekten in eine tiefkalte Flüssigkeit ist mit erheblichem Risiko verbunden, denn es kann auch bei sorgfältigem Arbeiten leicht zum Spritzen der Flüssigkeit kommen, was wiederum zu Verletzungen des Arbeiters führen kann. Außerdem ist ein unnötig hoher Verbrauch an Kühlmedium bei dieser Methode unvermeidlich.

Es ist daher Aufgabe der Erfindung, eine Vorrichtung zu schaffen, mit der auf einfache Weise ein tiefkaltes flüssiges Medium, wie z.B. flüssiger Stickstoff, gezielt und in dosierbarer Menge auf ein zu kühlendes Objekt, bspw. zu behandelnde Haut/Gewebeteile, schockzugefrierende kleine Objekte, zum Schrumpfen abzukühlende Werkstücke, in den Innenraum eines zu kühlenden Hohlkörpers, o.a., aufgebracht werden kann. Dabei soll die Handhabung des tiefkalten flüssigen Mediums einfach und sicher sein, und die Verdampfungsverluste beim Füllen und während der Benutzung sollen möglichst gering gehalten werden.

Diese Aufgabe wird erfindungsgemäß durch eine Pipette mit Vakuum-Isolierung gelöst. Durch die Vakuum-Isolierung können die Abdampfverluste sehr gering gehalten werden. Die Pipette ermöglicht außerdem bei einfachen Aufbau eine genaue Dosierung der abzugebenden Menge flüssigen Stickstoffs und auch die gezielte Abgabe auf engbegrenzte Flächen.

Die Verluste können weiter verringert werden, wenn nach einer vorteilhaften Ausführungsform der Erfindung der Außenmantel für die Vakuum-Isolierung innen ganz oder teilweise verspiegelt ist.

Zur einfachen Handhabung der erfindungsgemäßen Pipette sieht eine weitere vorteilhafte Ausgestaltung der Erfindung vor, daß im Bereich des oberen Endes ein umlaufender Wulst angeordnet ist. Dadurch kann die Pipette bei der Anwendung mit zwei Fingern gut gehalten

0060480

werden und der Daumen eine obere Öffnung zur dosierten Abgabe des flüssigen Stickstoffes aus einer unteren Öffnung ganz oder teilweise verschließen.

Für Anwendungsfälle, bei denen eine größere Menge flüssiger Stickstoff verwendet werden soll, wird der Aufnahmeraum der Pipette im Bereich des unteren Endes erweitert.

Um die Oberfläche des erweiterten Raumes bezogen auf das Volumen kleinzuhalten, kann die Erweiterung vorteilhaft als Hohlkugelraum ausgebildet sein.

Eine weitere Ausführungsform sieht vor, daß die Pipette am unteren Ende verschlossen ist und der Aufnahmeraum für die tiefkalte Flüssigkeit im oberen Bereich mit einem Auslaufstutzen versehen ist. Aus einer so ausgebildeten Pipette können dosierte Mengen Flüssigkeit auf das zu kühlende Objekt gegossen werden. In Ausgestaltung dieser vorteilhaften Ausführungsform sind im unteren Bereich des Aufnahmeraumes nach innen gerichtete Vorsprünge angeordnet, durch welche Siedesteine gehalten werden. Damit kann auf einfache Weise ein Siedeverzug im flüssigen tiefkalten Medium vermieden werden.

Bei einer weiteren Ausführungsform ist der Auslaufstutzen mit einem im Aufnahmeraum der Pipette angeordneten, bis nahezu an dessen Boden reichenden Steigrohr verbunden und parallel zum Füllstutzen noch oben gerichtet. Um zu vermeiden, daß in das Steigohr gelangende Dampfblasen die darüber befindliche Flüssigkeit aus dem Auslaufstutzen herausdrücken, ist das Steigrohr in Ausgestaltung dieser Ausführungsform an seinem oberen Ende vor der Einmündung in den Auslaufstutzen mit einer Erweiterung versehen. Diese bewirkt eine Trennung von Dampf und flüssiger Phase. In weiterer Ausgestaltung dieser Ausführungsform ist am Steigrohr oberhalb seiner Eintrittsöffnung eine scheibe aus Siedesteinmaterial angebracht. Damit wird Siedeverzug vermieden und es wird erreicht, daß die Siedeblasen in erster Linie oberhalb der Eintrittsöffnung des Steigrohrs entstehen, so daß nur vereinzelte Siedeblasen in das Steigrohr gelangen.

Um die Pipette in einfacher Weise standfest zu machen, wird sie mit einem Gehäuse umgeben. Sie wird damit zu einem einfach und sicher, ähnlich wie eine Spraydose zu handhabenden kleinen Vorratsbehälter für tiefkalte Flüssigkeit.

Bei einer zweckmäßigen Ausgestaltung ist der Auslaufstutzen der Pipette so ausgebildet, daß bekannte Injektionskanülen aufgesteckt werden können. Dies hat den Vorteil, daß die Form des Auslaufs durch Zurechtbiegen der Kanüle in einfachster Weise jeder speziellen Anwendung angepaßt und durch Wechseln der Kanüle verändert werden kann. Ferner ergibt sich in Weiterbildung dieser Ausführungsform die Möglichkeit, über einen an die Kanüle angeschlossenen Schlauch die Pipette mit einem Wärmetauscher zu verbinden. Dies hat den besonderen Vorteil, daß auch größere, mit dem Wärmetauscher in Kontakt gebrachte Flächen gezielt und wohldosiert mit flüssigem Stickstoff gekühlt werden können.

Der Verbrauch an flüssigem Stickstoff aus der Pipette läßt sich besonders vorteilhaft kontrollieren, wenn die Pipette in einem nicht verspiegelten Bereich (Sichtstreifen) der Wandfläche mit einer graduierten Skala versehen ist.

Es liegt im Rahmen der Erfindung, die untergeordneten Einzelmerkmale in unterschiedlicher Weise zu kombinieren.

Ausführungsbeispiele der Erfindung sind in den Zeichnungen dargestellt und werden im folgenden näher beschrieben:

Es zeigen in schematischer Darstellung und vorzugsweise im axialen Längsschnitt:

Fig.1    Eine einfache Ausführungsform der erfindungsgemäßen Pipette;

Fig.2    Eine Pipette nach Fig.1 mit erweitertem Aufnahmeraum;

Fig.3    Eine unten geschlossene Pipette nach der Erfindung mit seitlichem Auslaufstutzen im oberen Bereich;

Fig.4    Die Pipette nach Fig.3 in Anwendung;

Fig.5    Eine standfeste, mit einem Gehäuse umgebene Pipette mit Auslaufstutzen nach oben;

Fig.6 Die Pipette nach Fig.5 mit aufgesetzter Injektionskanüle und angeschlossenem Wärmetauscher.

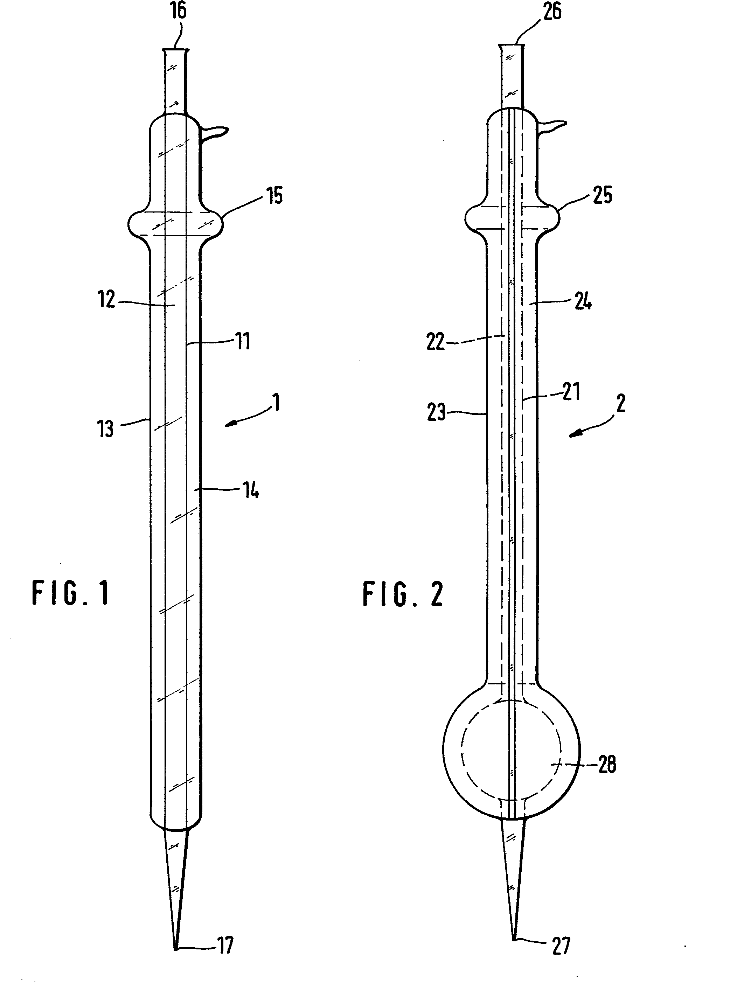

Die Pipette 1 (Fig.1) besteht aus einem langgestreckten,
unten spitz zulaufenden ersten Glasrohr 11, dessen
Innenraum den Aufnahmeraum 12 für flüssigen Stickstoff
bildet. Um den größten Teil des ersten Glasrohres 11
ist koaxial ein zweites Glasrohr 13 angeordnet, das
an seinem oberen und unteren Ende mit der Außenwand
des ersten Glasrohres 11 verschmolzen ist. Der Raum
zwischen der Außenwand des ersten Glasrohres 11 und
der Innenwand des zweiten Glasrohres 13 ist in bekannter
Weise evakuiert, wodurch der Aufnahmeraum 12 hervorragend wärmeisoliert wird. Ein umlaufender Wulst
15 im oberen Bereich des zweiten Glasrohres 13 dient
zum bequemen Halten der Pipette 1 zwischen Zeige- und
Mittelfinger, während mit dem Daumen die obere
Öffnung 16 des ersten Glasrohres 11 zum Halten des
flüssigen Stickstoffs verschlossen wird. Die untere
Auslauföffnung 17 hat einen sehr geringen Öffnungsdurchmesser, wodurch das Halten der Flüssigkeit begünstigt und die gezielte Abgabe durch Ausspritzen
(obere Öffnung 16 verschlossen) oder Auslaufen
(obere Öffnung 16 freigegeben) auf oder in das zu
kühlende Objekt erleichtert wird.

Bei der Pipette 2 (Fig.2), welche im übrigen gleich
der Pipette 1 aufgebaut ist, ist der Aufnahmeraum 22
im unteren Bereich als Kugelholraum 28 zur Vergrößerung
des Fassungsvermögens bei günstig kleiner Oberfläche ausgebildet. Die Innenwand des weiten äußeren Glasrohres
23 ist vom oberen Ende bis zum unteren Ende der hohlkugelförmigen Erweiterung verspiegelt, um die Wärmedämmung bezogen auf Strahlung noch zu verbessern. Selbstverständlich kann eine vollständige oder teilweise Verspiegelung bei allen Ausführungsformen der erfindungsgemäßen Vorrichtung durchgeführt werden. Um einen guten Überblick über den Füllstand der Pipette zu gewinnen, kann es jedoch vorteilhaft sein, in Längsrichtung über den gesamten Aufnahmeraum 22 auf eine Verspiegelung teilweise zu verzichten (Sichtstreifen).

Die Pipetten 1 und 2 zeichnen sich vor allem durch ihre Einfachheit aus. Die an sich vertraute Methode, Flüssigkeit aus einem Vorrat in kleinen Mengen zu entnehmen und an anderer Stelle abzugeben, wird durch die Anwendung der Vakuumisolation auch für tiefkalte Flüssigkeiten anwendbar gemacht. Diese Pipetten sind entsprechend ihrer einfachen Ausführung auch preiswert herzustellen. Eine gewisse Einschränkung der Anwendungsmöglichkeit ergibt sich daraus, daß die Haltezeit für die Flüssigkeit in der Pipette begrenzt ist. Auch bei Verspiegelung wird der Flüssigkeit in der Pipette nach und nach Wärme aus der Umgebung zugeführt, so daß Flüssigkeit verdampft und sich ein Überdruck in dem mit dem Daumen verschlossenen oberen Ende 16 des Glasrohres 11 ausbildet, der die Flüssigkeit nach einer gewissen Zeitspanne (je nach Ausbildung der Pipette 10 bis 20 Sekunden) aus der unteren Öffnung 17 heraustreibt. Für viele Anwendungen ist diese Zeitspanne zur Handhabung jedoch vollständig ausreichend. Wird die Entleerung der Pipette in noch kürzerer Zeit gewünscht, braucht nur die obere Öffnung 16 des Rohres 11 freigegeben zu werden.

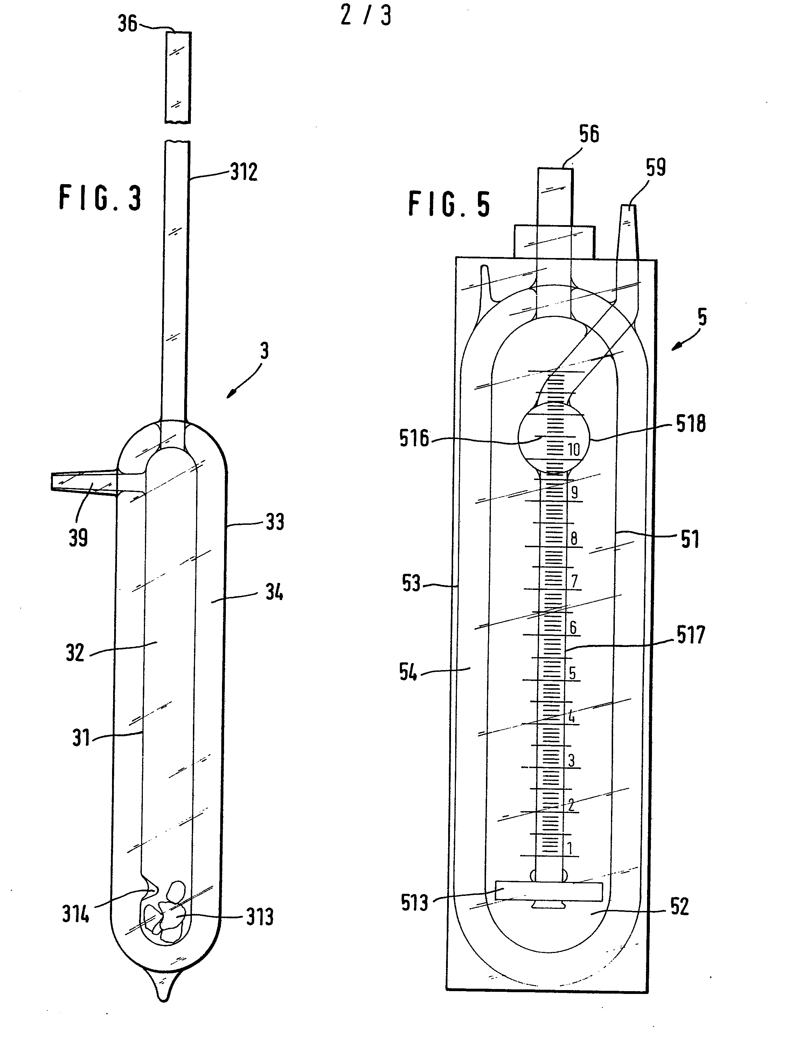

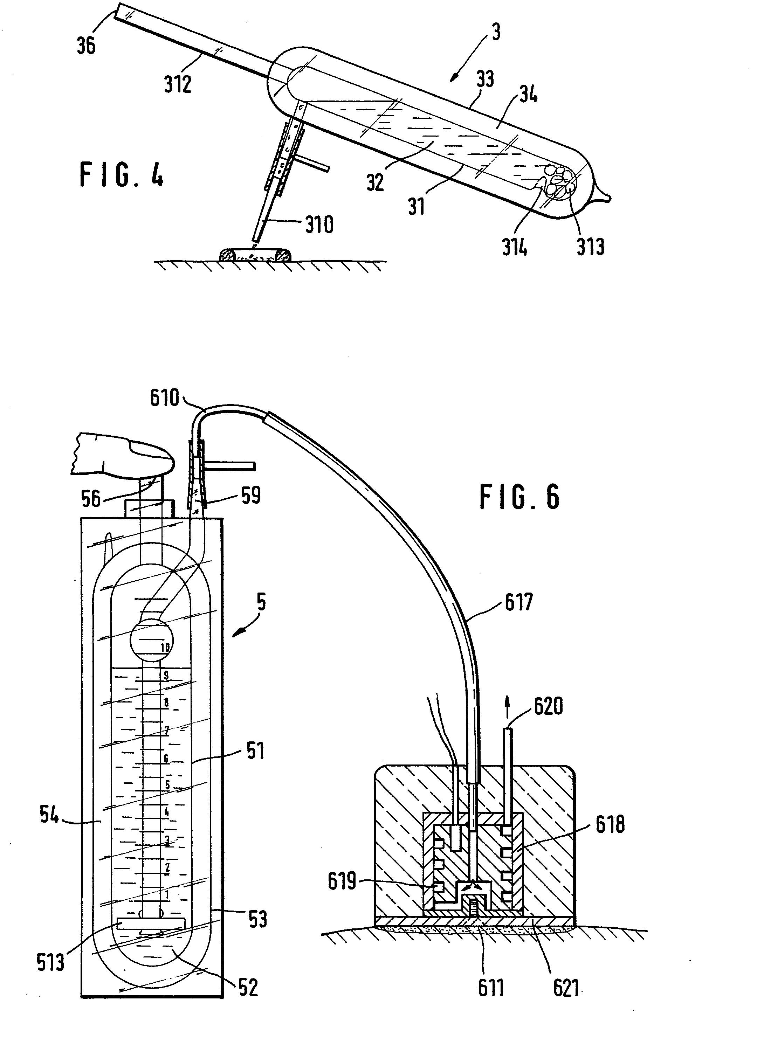

Die Pipette 3 (Fig.3) besteht wieder aus einem ersten, innenliegenden Glasrohr 31 mit dem Aufnahmeraum 32 für die Flüssigkeit, welcher von einem zweiten, äußeren Glasrohr 33 konzentrisch umgeben ist. Bei dieser Ausführungsform ist jedoch das erste Glasrohr 31 und damit der Aufnahmeraum 32 unten verschlossen. Der Auslauf ist als Stutzen 39 seitlich herausgeführt und konisch ausgebildet, so daß eine handelsübliche Injektionskanüle 310 zur Verlängerung des Auslaufs, Formgebung für den Auslauf entsprechend der speziellen Anwendung, oder auch Kupplungselement aufgesetzt werden kann, wie aus der einen Anwendungsfall veranschaulichenden Fig. 4 ersichtlich ist. Wie Fig.4 zeigt, kann der flüssige Stickstoff aus der Pipette durch Schräghalten derselben aus dem Auslauf 39,310 auf ein zu kühlendes Werkstück oder auf eine zu gefrierende Haut/Gewebepartie, bspw. zur Behandlung von Warzen, aufgetropft werden. Die austropfende oder auslaufende Flüssigkeitsmenge kann in einfacher Weise durch Variation der Schräglage der Pipette verändert werden. Durch Aufrichten der Pipette wird die Flüssigkeitsabgabe unterbrochen. Für bestimmte Anwendungen kann es vorteilhaft sein, eine bestimmte Fläche auf dem zu kühlenden Objekt wie in Fig. 4 gezeigt durch einen Rand, bspw. aus einem plastischen Kitt, abzugrenzen, wodurch die Verbreitung der Flüssigkeit auf dem Objekt auf einen bestimmten Bereich beschränkt wird

Die Pipette 3 kann aber auch benutzt werden, um Flüssigkeit auf oder in ein zu kühlendes Objekt zu spritzen. Dazu wird die obere Öffnung 36 wie bei den Pipetten 1 und 2 mit einem Finger verschlossen, wodurch sich während der Schräglage der Druck über erhöht, so daß sie aus der Pipette herausgetrieben wird. Die pro Zeiteinheit austretende Menge kann so merklich erhöht werden. Außerdem kann durch Öffnen und Wieder- verschließen der Öffnung 36 mit dem Finger die austretende Flüssigkeitsmenge ohne jedes Ventil in einfachster Weise sehr genau dosiert werden.

Die Haltezeit der Pipette 3 für flüssigen Stickstoff liegt der Größenordnung von 10 bis 40 Minuten, je nach Volumen des Aufnahmeraumes und Art der Vakuumisolierung (unverspiegelt, verspiegelt). Zur Vermeidung von Siedeverzug im flüssigen Stickstoff sind bei der Pipette 3 im unteren Teil des Aufnahmeraums 32 Siedesteine 313 angeordnet, die durch nach innen gerichtete Ausbeulungen 314 im inneren Glasrohr 31 an ihrem Platz gehalten werden.

In Fig. 5 ist eine Pipette 5 dargestellt, die von einem Gehäuse mit flachem Boden umgeben und damit standfest ist. Im Aufnahmeraum 52 dieser Pipette ist ein fast bis an dessen Boden reichendes Steigrohr 517 angeordnet, das in seinem oberen Teil mit einer kugelförmigen Erweiterung 518 versehen und an den hier nach oben gerichteten, parallel zum oberen, als Füllstutzen dienenden Ende 56 des Innenrohrs 51 angeordneten Auslaufstutzen 59 angeschlossen ist. Am unteren Ende des Steigrohrs 517 ist oberhalb seiner Eintrittsöffnung eine Scheibe 513 aus Siedesteinmaterial angebracht. Mit dieser Anordnung wird erreicht, daß sich die Mehrzahl der Siedeblasen an der Scheibe 513 oberhalb der Eintrittsöffnung des Steigrohrs 517 bildet, also nicht in das Steigrohr 517 gelangt. Einzelne Siedeblasen, die dennoch im Steigrohr 517 anfallen, werden durch die als Phasentrenner wirkende Erweiterung 518 daran gehindert, Flüssigkeit aus dem Auslaufstutzen 59 herauszutreiben. In der Erweiterung 518 trennen sich Flüssigkeit und Dampf und das sogenannte "Blasenpumpen" wird wirkungsvoll unterdrückt. An der Wandung des inneren oder äußeren Glasrohrs (51 oder 53) ist bei der Pipette 5 eine Gradskala 516 angebracht, die dazu dient, die Füllhöhe bzw die verbrauchte Menge an flüssigem Stickstoff präzise abzulesen. Bei Verspiegelung wird in diesem Bereich ein Sichtstreifen ausgespart. Die Standzeit der Pipette 5 entspricht derjenigen der Pipette 4.

Die Pipette 5 wird nach der Spritzmethode angewendet, d.h. der Füllstutzen 56 wird mit dem Finger verschlossen, wodurch sich über der Flüssigkeit Druck aufbaut, so daß Flüssigkeit über das Steigrohr 517 aus dem Auslaufstutzen 59 herausgetrieben wird. Die Regulierung der Flüssigkeitsmenge erfolgt wieder sehr genau durch Schließen oder Freigeben der Öffnung 56. Diese Pipette braucht nicht schräggelegt zu werden. Sie wird gehalten, wie eine mit Treibmittel und einem zu versprühenden Medium gefüllte Sprayflasche und auch die Betätigung ist entsprechend, nur wird anstatt Betätigung eines niederzudrückenden Ventilknopfes hier lediglich die Öffnung 56 mit dem Finger verschlossen oder freigegeben. Um die austretende Flüssigkeit gezielt aufbringen zu können, wird auf den Auslaufstutzen 59 entweder nur eine entsprechend zurechtgebogene Injektionskanüle aufgesetzt, oder es wird direkt oder mittels einer Injektionskanüle ein dünner Schlauch angeschlossen (bspw. Paragummi, der nicht brüchig wird), der auf das zu kühlende Objekt gerichtet werden kann. Mit dieser Pipette können auch größere Flächen gezielt mit Stickstoff besprüht werden.

Eine besondere Verwendungsform dieser Pipette 5 ist in Fig.6 dargestellt. Der Auslaufstutzen 39 ist über eine Kanüle 610 und einen Gummischlauch 617 an einen Wärmetauscher 618 angeschlossen. Dieser besteht aus einem Metallkörper, der mit Hohlräumen 619 versehen ist, die von Stickstoff durchströmt werden, welcher durch den offenen Stutzen 620 abdampft. Der Wärmetauscher 618 ist hier auf einer Kühlplatte 621 angeordnet, die über einzufrierendes Gewebe 611 gelegt ist. Er kann nach außen in üblicher Weise isoliert sein und die Möglichkeit für den Anschluß beispielsweise eines Temperaturmeßfühlers bieten. Die Kombination der Pipette 5 mit einem Wärmetauscher 618 gibt die Möglichkeit, größere Flächen auch nachhaltiger und mit größerer Tiefenwirkung dosiert zu kühlen, als es durch Ansprühen mit flüssigem Stickstoff möglich ist.

Den erfindungsgemäßen Pipetten ist unabhängig von der Ausführungsform gemeinsam, daß sie einfach aufgebaut und damit preiswert in der Herstellung sowie einfach und zuverlässig in der Handhabung sind. Sie enthalten keinerlei mechanische Teile, bspw. Ventile, die verstopfen oder versagen könnten. Ferner ist das für manche Anwendungen benötigte Zubehör größtenteils standardmäßig verfügbar ( handelsübliche medizinische Injektionskanülen, Fahrrad-Ventilschlauch) und äußerst preiswert. Die Handhabung und Dosierung von flüssigem Stickstoff in kleinen Mengen in der Fertigungstechnik oder in der Medizin wird damit einfach, auch für unge- übtes Personal verständlich, zuverlässig und sicher.

Gefüllt werden die Pipetten entweder durch Eintauchen in ein mit flüssigem Stickstoff gefülltes Vorratsgefäß oder über einen in einen Vorratsbehälter eingesetzten Heber mit Ventil. Vorratsgefäß, Vorratsbehälter und Heber sind ebenfalls handelsübliche Standardteile.

Patentansprüche

1. Vorrichtung zur Handhabung und Dosierung kleiner
   Mengen tiefkalten flüssigen Mediums, vorzugsweise
   flüssigen Stickstoffs,
   gekennzeichnet durch eine Pipette (1,2,3,5) mit
   Vakuum-Isolierung.

2. Vorrichtung nach Anspruch 1,
   dadurch gekennzeichnet, daß der Außenmantel (13,23,
   33,53) für die Vakuum-Isolierung innen ganz oder
   teilweise verspiegelt ist.

3. Vorrichtung nach Anspruch 1 und/oder 2,
   dadurch gekennzeichnet, daß die Pipette (1,2) im
   Bereich des oberen Endes einen umlaufenden Wulst
   (15,25) aufweist.

4. Vorrichtung nach Anspruch 1,2 und/oder 3,
   dadurch gekennzeichnet, daß der Aufnahmeraum (12,22)
   der Pipette für die tiefkalte Flüssigkeit im
   Bereich des unteren Endes erweitert ist.

5. Vorrichtung nach Anspruch 4,
   dadurch gekennzeichnet, daß die Erweiterung als
   Hohlkugelraum (28) ausgebildet ist.

6. Vorrichtung nach Anspruch 1, 2 und/ oder 3,
   dadurch gekennzeichnet, daß am oberen Ende des
   Aufnahmeraumes (32, 52) einer unten verschlossenen
   Pipette (3, 5) ein Auslaufstutzen (39, 59 angebracht
   ist.

7. Vorrichtung nach Anspruch 6, dadurch gekennzeichnet, daß im unteren Bereich des Aufnahmeraumes ( 32) für den flüssigen Stickstoff nach innen gerichtete Vorsprünge ( 314) angeordnet sind, durch welche Siedesteine ( 313) gehalten werden.

8. Vorrichtung nach Anspruch 6 und/oder 7, dadurch gekennzeichnet, daß die Pipette ( 5) in einem Gehäuse ( 515) angeordnet ist und der Austrittsstutzen ( 59) nach oben weist.

9. Vorrichtung nach Anspruch 8, gekennzeichnet durch eine Verwendung der Pipette ( 5) als Vorratsbehälter für flüssigen Stickstoff zum Anschluß an einen Wärmetauscher ( 618).

10. Vorrichtung nach einem der Ansprüche 1 bis 9, dadurch gekennzeichnet, daß die Pipette ( 1, 2, 3, 5) im nichtverspiegelten Bereich mit einer graduierten Skala ( 516) versehen ist.

FIG. 1

FIG. 2

FIG. 3

36

312

3

39

33

34

32

31

314

313

FIG. 5

56

59

5

516

518

51

53

54

517

513

52

FIG. 4

FIG. 6

Europäisches Patentamt

**EUROPÄISCHER RECHERCHENBERICHT**

0060480

EP 82 10 1819

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl. 3) |
|---|---|---|---|
| A | DE-A-2 534 705 (BAUER) *Ansprüche 1,7,8; Abbildungen 5,10* | 1,2 | A 61 B 17/36 B 01 L 3/02 |
| A | GB-A- 618 628 (CONICK) *Abbildungen 1,2* | 3 | |
| A | GB-A- 651 374 (POSTANS) *Seite 2, Zeilen 46-54; Abbildungen 1,2* | 4 | |
| A | US-A-3 651 813 (BRYNE) *Spalte 4, Zeilen 22-42; Abbildungen 1,2* | 6,8 | |
| A | US-A-3 823 718 (TROMOVITCH) *Spalte 2, Zeile 4 - Spalte 3, Zeile 25; Abbildungen 1-3* | 1,6,8 | RECHERCHIERTE SACHGEBIETE (Int. Cl. 3) |
| A | DE-A-1 541 181 (KATZ) *Spalte 2, Zeilen 1-38;Abbildung 1* | 1 | A 61 B B 01 L B 01 J |
| A | CA-A- 919 926 (ZELLERMAN) *Seite 2, Zeile 27 - Seite 4, Zeile 12; Abbildung 1* | 1,2 | |

Der vorliegende Recherchenbericht wurde für alle Patentansprüche erstellt.

| Recherchenort | Abschlußdatum der Recherche | Prüfer |
|---|---|---|
| DEN HAAG | 22-06-1982 | BARTLETT S.C |